# EUROPÄISCHE PATENTSCHRIFT

(11) **EP 2 880 183 B1**
(45) Veröffentlichungstag und Bekanntmachung des Hinweises auf die Patenterteilung: **11.04.2018**
(21) Anmeldenummer: 13771414.3
(22) Anmeldetag: 18.09.2013
(51) Int. Cl.: C12Q 1/68, G01N 33/487, B81B 1/00

(54) **ANORDNUNG ZUR NUKLEINSÄURE-SEQUENZIERUNG MITTELS TUNNELSTROMANALYSE**
ASSEMBLY FOR NUCLEIC ACID SEQUENCING BY MEANS OF TUNNEL CURRENT ANALYSIS
SYSTÈME POUR LE SÉQUENÇAGE D'ACIDES NUCLÉIQUES PAR ANALYSE PAR COURANT TUNNEL

(30) Priorität: 27.09.2012 DE 102012217603
(43) Veröffentlichungstag der Anmeldung: 10.06.2015
(73) Patentinhaber: Siemens Healthcare GmbH, 91052 Erlangen (DE)
(72) Erfinder: GUMBRECHT, Walter, 91074 Herzogenaurach (DE); HAYDEN, Oliver, 91074 Herzogenaurach (DE)
(86) Internationale Anmeldenummer: PCT/EP2013/069384
(87) Internationale Veröffentlichungsnummer: WO 2014/048816

(56) Entgegenhaltungen:
- EP-A1- 1 657 539
- WO-A1-2011/097171
- WO-A2-02/074984
- WO-A2-2008/071982
- US-A1- 2006 019 259
- US-A1- 2012 193 231
- US-A1- 2012 193 236
- IVANOV ALEKSANDAR P ET AL: "DNA tunneling detector embedded in a nanopore.", NANO LETTERS 12 JAN 2011, Bd. 11, Nr. 1, 12. Januar 2011 (2011-01-12), Seiten 279-285, XP002715875, ISSN: 1530-6992
- ALBRECHT TIM: "How to understand and interpret current flow in nanopore/electrode devices.", ACS NANO 23 AUG 2011, Bd. 5, Nr. 8, 23. August 2011 (2011-08-23) , Seiten 6714-6725, XP002715876, ISSN: 1936-086X

## Beschreibung

Die Erfindung betrifft eine Anordnung zur Nukleinsäure-Sequenzierung mittels Tunnelstromanalyse.

In der Literatur ist eine Vielzahl von Methoden zur Nukleinsäure-Sequenzierung bekannt. Dazu gehören Verfahren der sogenannten Sequenzierung durch Synthese (englisch: Sequencing-by-Synthesis). Hier wird bei Einbau eines passenden Nukleotids eine Komponente freigesetzt, die mittels Enzymkaskaden nachgewiesen wird. Weiterhin ist die Nukleinsäure-Sequenzierung in Nanoporen bekannt. Von Vorteil ist, dass bei dieser Methode weder eine Markierung des DNA-Strangs noch eine komplexe Reaktionskaskade benötigt wird.

Bei der Nukleinsäure-Sequenzierung mittels Nanoporen passieren DNA-Stränge eine biologische oder künstliche (engl. Solid-State) Nanopore. Einzelne Basen des Nukleinsäurestrangs können durch eine Veränderung des Porenwiderstands beim Passieren der DNA durch die Nanopore ananalysiert werden. Die DNA wird dabei in ein leitendes Fluid gegeben. An das Fluid wird eine Spannung angelegt, so dass ein elektrischer Strom fließt. Beim Passieren von unterschiedlichen Basenarten (insbesondere Guanin, Cytosin, Thymin, Adenin) durch die Nanopore ändert sich dieser Strom. Diese Änderung ist abhängig von der Base, welche die Pore passiert, so dass die Base analysiert werden kann.

Alternativ kann ein Tunnelstrom über die Pore gemessen werden (englisch: sequencing-by-tunneling), wobei der Tunnelstrom abhängig ist von der Base, welche sich in der Pore befindet. Tunnelstromverfahren haben vorteilhafterweise eine bessere Basenauflösung im Vergleich zur Messung des Porenwiderstands. Dieser begründet sich in hohen elektrischen Feldstärken innerhalb der Nanopore.

Diese Messungen hängen stark von der Größe und Form der Nanopore ab. Weiterhin müssen die Elektroden zum Anlegen einer Spannung an die Nanopore exakt angeordnet werden, um den Tunnelstrom ausreichend genau zu erfassen. Um eine hohe Analysesicherheit der Nukleinsäure-Sequenzierung mittels Tunnelstromanalyse zu erreichen, ist es wünschenswert, maßgeschneiderte Nanoporen mit Elektroden für definierte Anwendungen herzustellen. Die meisten Herstellungsverfahren zur Erzeugung von solid-state Nanoporen basieren auf dem Entfernen von Material aus einer dünnen Membran, ähnlich dem Bohren von Löchern. Diese Herstellung wird insbesondere mittels elektronenstrahlbasierter Fotolithografie durchgeführt.

WO 2008/071982 A2, WO 2011/097171 A1 und US 2012/193236 A1 beschreiben jeweils Vorrichtungen zur Tunnelstromanalyse, sowie Verfahren zu deren Herstellung und erwähnen die Wichtigkeit der präzisen Anordnung der Nanopore und beschreiben allesamt sehr zeit- und arbeitsaufwendige Verfahren, zur Herstellung einer Tunnelstromanalysevorrichtung.
Die bisherigen Anordnungen von solid-state Nanoporen mit Elektroden sind nachteiligerweise sehr komplex und zeitaufwändig in der Herstellung. Eine ausreichend präzise Anordnung der Nanopore zu den Elektroden oder einem Kondensator derart, dass Tunnelströme mit hoher Genauigkeit gemessen werden können, ist derzeit kaum technisch verwirklichbar und deren Herstellung ist nachteiligerweise mit hohem Arbeits- und Zeitaufwand verbunden.

Aufgabe der vorliegenden Erfindung ist es, eine Anordnung zur Analyse von Nukleinsäuresequenzen und ein Verfahren zum Herstellen der Anordnung anzugeben, welche die genannten Nachteile überwinden.

Die Aufgabe wird mit der in Anspruch 1 angegebenen Anordnung und dem in Anspruch 11 angegebenen Verfahren gelöst. Die abhängigen Ansprüche betreffen vorteilhafte Weiterbildungen und Ausgestaltungen der Erfindung.

Die Anordnung zur Nukleinsäure-Sequenzierung mittels Tunnelstromanalyse umfasst erfindungsgemäß wenigstens zwei elektrisch leitfähige Partikel mit einem Durchmesser von 1 nm bis 100 nm, insbesondere mit einem Durchmesser von 1 nm bis 10 nm. Weiterhin umfasst sie wenigstens zwei elektrisch isolierende Partikel mit einem Durchmesser von 1 nm bis 100 nm, insbesondere von 1 nm bis 10 nm. Die Anordnung umfasst weiterhin wenigstens zwei erste Elektroden zum Kontaktieren der elektrisch leitfähigen Partikel. Die ersten Elektroden und die Partikel befinden sich auf einem Substrat. Erfindungsgemäß sind die wenigstens vier Partikel im Wesentlichen quadratisch planar angeordnet, wobei sich die leitfähigen Partikel und isolierenden Partikel jeweils diagonal gegenüberliegen. Vorteilhaft bildet sich in der Mitte der vier quadratisch planar angeordneten Partikel eine Lücke aus. Die Größe dieser Lücke liegt im Bereich von nm in Abhängigkeit der Größe und Form der Partikel. Die Lücke stellt bei der Analyse von Nukleinsäuresequenzen eine solid-state Nanopore dar. Die Anordnung ermöglicht es, eine definierte maßgeschneiderte Nanopore darzustellen.

Bei dem erfindungsgemäßen Verfahren zum Herstellen einer Anordnung zur Nukleinsäure-Sequenzierung mittels Tunnelstromanalyse werden Vertiefungen in einem Substrat erzeugt. Es werden wenigstens zwei elektrisch leitfähige Partikel und wenigstens zwei elektrisch isolierenden Partikeln in die Vertiefungen gegeben, insbesondere gefüllt, wobei die Partikel in den Vertiefungen statistisch verteilt werden.

In einer vorteilhaften Ausgestaltung und Weiterbildung der Erfindung stehen die ersten Elektroden jeweils mit wenigstens einem elektrisch leitenden Partikel in direktem elektrischem Kontakt. Vorteilhaft kann so ein Strom durch die elektrisch leitenden Partikel fließen. Die Anordnung der ersten Elektroden ist vorteilhaft derart, dass die leitfähigen Partikel den Kondensator bilden, an dem ein Tunnelstrom zur Analyse gemessen werden kann, wenn sich ein DNA- oder RNA-Strang in der Nanopore befindet. Die Anordnung der Nanopore zu dem Kondensator ist dann vorteilhafterweise derart, dass Tunnelströme mit hoher Genauigkeit gemessen werden können, da Nanopore und Kondensator örtlich sehr dicht aneinander liegen. Im Idealfall sind der sich bildende Kondesatorspalt und die Nanopore identisch.

In einer weiteren vorteilhaften Ausgestaltung und Weiterbildung der Erfindung umfasst die Anordnung wenigstens zwei orthogonal zu den ersten Elektroden angeordnete zweite Elektroden. Diese Elektroden dienen vorteilhaft dem zielgerichteten Bewegen eines DNA-Strangs durch die Partikel. Insbesondere eignen sich hierfür Elektroden, die bei der Gelelektrophorese eingesetzt werden, um die DNA/RNA durch das Gel zu bewegen.

In einer weiteren vorteilhaften Ausgestaltung und Weiterbildung der Erfindung umfasst das Substrat im Raster angeordnete Vertiefungen. Der Durchmesser der Vertiefung liegt insbesondere zwischen 10 nm und 1 µm. Vorteilhaft stellen die Vertiefungen eine Fixierungseinheit für die Partikel dar.

In einer weiteren vorteilhaften Ausgestaltung und Weiterbildung der Erfindung ist das Substrat ein CMOS-Chip. Dieser ist in Schichten aufgebaut, wobei isolierende und leitende Schichten übereinander angeordnet sind. Der CMOS-Chip umfasst typischerweise bereits eine Vorrichtung zur Spannungsversorgung der Elektroden sowie zum Messen eines Stroms zwischen den ersten Elektroden.

Die Partikel sind sphärische Partikel. Die sphärischen Partikel ordnen sich in einer Kugelpackung an. Die Durchmesser der so entstehenden Lücken lassen sich vorteilhaft berechnen. Maßgeschneiderte Nanoporen zur Nukleinsäure-Sequenzierung können somit ausgebildet werden.

In einer weiteren vorteilhaften Ausgestaltung und Weiterbildung der Erfindung umfassen die elektrisch leitfähigen Partikel Gold.

Die elektrisch leitfähigen Partikel weisen im Wesentlichen dieselbe Größe auf. Vorteilhaft ist die Kugelpackung dadurch regelmäßig.

In einer weiteren vorteilhaften Ausgestaltung und Weiterbildung der Erfindung umfassen die elektrisch isolierenden Partikel Polystyrol.

In einer weiteren vorteilhaften Ausgestaltung und Weiterbildung der Erfindung sind die elektrisch leitfähigen Partikel fest miteinander verbunden. Diese feste Verbindung kann vorteilhaft mit Hilfe eines Galvanisierungsprozesses erzeugt werden. Alternativ kann diese feste Verbindung mittels einer elektrisch leitfähigen Beschichtung, welche insbesondere mit Hilfe des stromlosen Abscheidens von Metall erzeugt wird, erfolgen. Der elektrische Kontakt zwischen den elektrisch leitenden Partikeln, welche jeweils weniger als 1nm voneinander entfernt sind, wird somit vorteilhaft sichergestellt.

In einer vorteilhaften Ausgestaltung und Weiterbildung der Erfindung werden die Vertiefungen mit wenigsten 100 elektrisch leitenden und elektrisch isolierenden Partikeln befüllt.

In einer vorteilhaften Ausgestaltung und Weiterbildung der Erfindung werden die Vertiefungen, welche bereits mit elektrisch leitenden und elektrisch isolierenden Partikeln befüllt sind selektiert, welche genau eine Anordnung gemäß Anspruch 1, also mit einer Nanopore, aufweisen. Vorteilhafterweise wird der Tunnelstrom, welcher bei Durchgang einer DNA/RNA durch die Nanopore fließt, ohne die Überlagerung weiterer Tunnelströme gemessen.

In einer weiteren vorteilhaften Ausgestaltung und Weiterbildung der Erfindung werden die elektrisch leitfähigen Partikel, welche zueinander weniger als 1nm Abstand haben, mit einer elektrisch leitfähigen Schicht derart beschichtet, dass sie in elektrischem Kontakt miteinander stehen. Der elektrische Kontakt zwischen den elektrisch leitenden Partikeln, welche jeweils weniger als 1nm voneinander entfernt sind, wird somit vorteilhaft sichergestellt.

In einer weiteren vorteilhaften Ausgestaltung und Weiterbildung der Erfindung werden die elektrisch leitfähigen Partikel mittels stromlosen Abscheidens von Metall beschichtet.

Die Erfindung wird nachfolgend an Hand eines Ausführungsbeispiels näher erläutert.
Figur 1 zeigt schematisch eine Partikelanordnung 13 in Draufsicht von oben.
Figur 2 zeigt schematisch den Aufbau der Rasteranordnung 1 von der Seite. Die Rasteranordnung 1 umfasst die Partikelanordnung 13.
Figur 3 zeigt schematisch eine Vertiefung 3 mit zwei ersten Elektroden 4 in Draufsicht von oben mit einem elektrischen Strom 10.

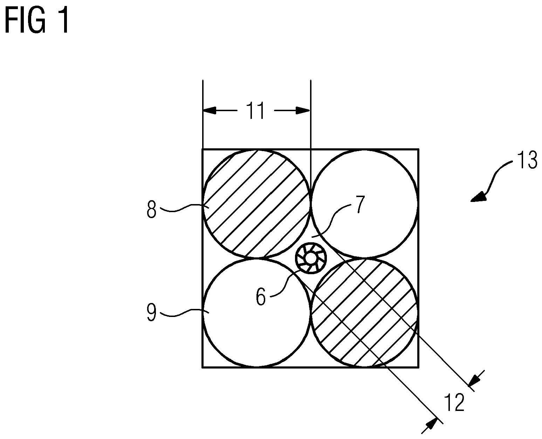

Die in Figur 1 schematisch dargestellte Partikelanordnung 13 umfasst zwei leitende Partikel 8, zwei isolierende Partikel 9, und DNA 6. Die Partikel sind im Schnitt in Draufsicht von oben dargestellt, das heißt die dargestellten Runden Flächen zeigen die Schnittfläche eines Partikels bei dessen maximalen Durchmesser. Auch die helixförmige DNA 6 ist von oben zu sehen.

Die leitenden Partikel 8 bestehen aus Gold. Die isolierenden Partikel 9 bestehen aus Polystyrol. Alternativ bestehen die isolierenden Partikel 9 aus Latex. Der Durchmesser 11 der Partikel 8 und 9 beträgt 5 nm. Die Lücke, welche sich zwischen den Partikeln ausbildet, stellt eine aktive Nanopore 7 für eine DNA-Sequenzierung dar. Sie hat einen Porendurchmesser 12 von 2 nm.

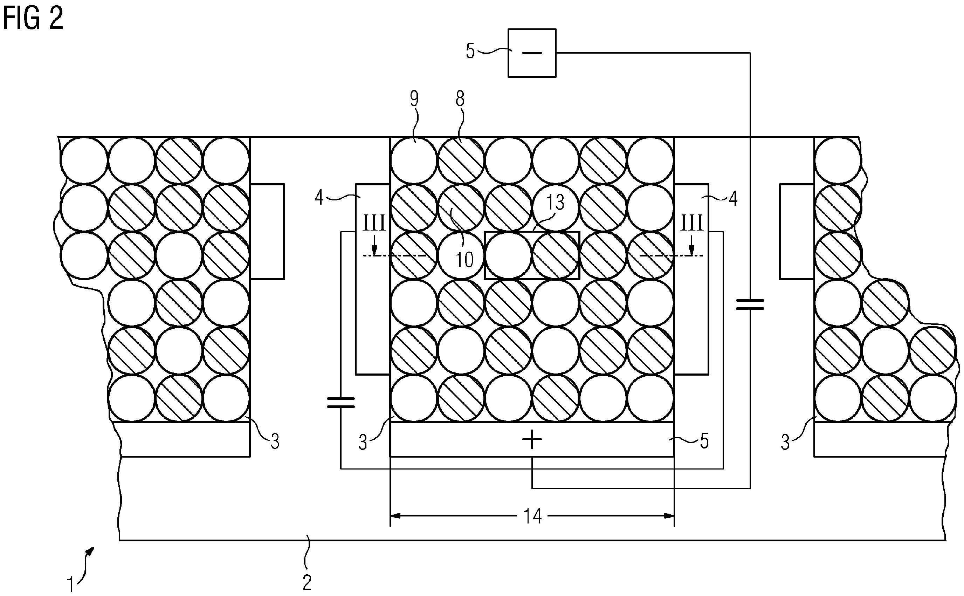

Figur 2 zeigt eine Seitenansicht der Rasteranordnung 1 mit beispielhaft drei Vertiefungen 3. Das Substrat 2 umfasst insbesondere Silizium. Der Vertiefungsdurchmesser 14 beträgt typischerweise 30-150 nm. Der Vertiefungsdurchmesser 14 in diesem Ausführungsbeispiel beträgt 35 nm. An zwei der seitlichen Wände der Vertiefung 3 befindet sich je eine erste Elektrode 4. Die ersten Elektroden 4 werden zweckmäßigerweise mit Spannung versorgt. Die ersten Elektroden 4 können sich, wie in diesem Beispiel, über Teile der seitlichen Wände erstrecken. Alternativ können die ersten Elektroden 4 sich auch über die gesamte Fläche der seitlichen Wände erstrecken. Am Boden der Vertiefung 3 und oberhalb des Substrats 2 ist jeweils eine zweite Elektrode 5 angeordnet. Die Spannungsversorgung der Elektroden 4 und 5 ist beispielhaft anhand der mittleren Vertiefung 3 der Figur 2 dargestellt.

Das Substrat ist typischerweise ein CMOS-Chip. Auf diesem CMOS-Chip werden die Vertiefungen im Raster angeordnet. Eine der zweiten Elektroden 5 und die beiden ersten Elektroden 4 sind auf diesem Substrat, d. h. dem CMOS-Chip, aufgebracht. Der CMOS-Chip weist sehr gute analog-elektronische Eigenschaften auf, welche eine präzise Messung des Tunnelstroms gewährleisen. Insbesondere über eine Analog-Digital-Wandlung und ein schnelles Multiplexverfahren ist es möglich, eine Vielzahl von Elektroden zum Messen des Tunnelstroms auszulesen. Die Kontaktierung der Elektroden erfolgt insbesondere mittels der oberste Metallisierungsebene des CMOS-Chips.

Die Vertiefungen 3 werden mittels einer Ätztechnik in Siliziumoxid oder Siliziumnitrid eingebracht.

Die Vertiefung 3 ist mit einem Gemisch aus leitenden Partikeln 8 und isolierenden Partikeln 9 befüllt, wobei die Zusammensetzung des Gemischs im Wesentlichen hälftig aufgeteilt ist und sich die Partikel statistisch in der Vertiefung verteilen.

Die zweiten Elektroden 5 werden mit Spannung versorgt, so dass die DNA 6 in die Vertiefung 3 hinein transportiert wird, ähnlich dem Transport der DNA in einer Elektrophoreseanordnung.

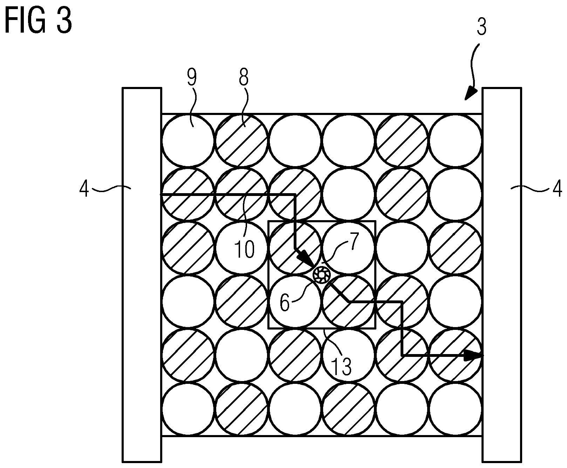

In Figur 3 ist Schnitt durch eine der Vertiefungen 3 dargestellt. In der aktiven Nanopore 7 befindet sich eine DNA 6. Wird Spannung an die ersten Elektroden 4 angelegt, so entsteht in der Partikelanordnung 13 ein elektrischer Strom 10. Zwischen den leitenden Partikeln 8 fließt ein Tunnelstrom, wobei die leitenden Partikel 8 selber die Kondensatoren darstellen. Der Tunnelstrom ist abhängig von der Base der DNA 6, welche die aktive Nanopore 7 passiert. Mit Hilfe einer Messung des Tunnelstroms zwischen den beiden ersten Elektroden 4 kann somit die Base in der aktiven Nanopore 7 analysiert werden.

Vorteilhaft bilden die leitenden Partikel 8, welche direkt an die aktive Nanopore 7 grenzen, den Kondensator, was eine kurze Porenlänge ermöglicht. Dies verbessert die Basenauflösung der DNA-Sequenzierung deutlich.

Der Strom zwischen den zweiten Elektroden 5 bewegt die DNA 6 durch die aktive Nanopore 7, so dass eine Base nach der anderen die aktive Nanopore 7 passiert und die Nukleinsäure-Sequenz analysiert wird. Die zweiten Elektroden 5 werden während der Messung des Tunnelstroms nicht mit Spannung versorgt. Alternativ kann eine konstante Spannungsversorgung der zweiten Elektroden 5 erfolgen, falls diese die Messung des Tunnelstroms nicht stört.

Um geeignete Systeme, insbesondere Partikelanordnungen 13, mit aktiven Nanoporen 7 zu adressieren, werden die Vertiefungen 3 zunächst mit einer Elektrolytlösung befüllt. Anschließend wird eine Wechselspannung an die mit Partikeln und Elektrolytlösung befüllte Vertiefung 3 angelegt und der Widerstand gemessen. Der Widerstand ist charakteristisch für die Anordnung der nichtleitenden und leitenden Partikel 8 und 9.

Falls eine Vertiefung 3 mehrere Partikelanordnungen 13 umfasst, in denen sich zeitgleich DNA 6 befindet, so führt dies zu mehreren Tunnelströmen in einer Vertiefung 3. Diese Tunnelströme lassen sich dann nicht diskriminieren, so dass diese Vertiefung 3 nicht ausgewertet werden kann. Ein Raster umfasst in diesem Beispiel allerdings 1000 Vertiefungen 3, so dass dennoch ausreichend Vertiefungen 3 mit genau einer Partikelanordnung 13 zur DNA-Analyse zur Verfügung stehen.

Zur Fixierung der Partikel kann eine Galvanisierung erfolgen. Die leitenden Partikel 8 aus Gold werden durch sogenannte stromlose Galvanisierung miteinander verbunden. Alternativ kann diese Kontaktierung echt galvanisch geschehen. Die leitenden Partikel werden somit fixiert und ein elektrischer Kontakt zwischen den sich direkt berührenden leitenden Partikeln 8 sichergestellt.

Alternativ zu DNA-Sequenzen können auch RNA-Sequenzen analysiert werden. Auch die Sequenz-Analyse kurzer RNA-Fragmente, insbesondere von miRNAs (micro-RNAs) oder mRNAs (messenger RNA), ist möglich.

## Patentansprüche

1. Anordnung zur Nukleinsäure-Sequenzierung mittels Tunnelstromanalyse umfassend:
- wenigstens zwei elektrisch leitfähige Partikel (8) mit einem Durchmesser (11) von 1 nm bis 100 nm,
- wenigstens zwei elektrisch isolierende Partikel (9) mit einem Durchmesser von 1 nm bis 100 nm,
- wenigstens zwei erste Elektroden (4) zum Kontaktieren der elektrisch leitfähigen Partikel (8),
- ein Substrat (2) auf dem die ersten Elektroden (8) und die Partikel (8,9) angeordnet sind,
- wobei die vier Partikel (8,9) im Wesentlichen quadratisch planar angeordnet sind und sich die leitfähigen Partikel (8) und die isolierenden Partikel (9) jeweils diagonal gegenüber liegen,
- wobei die Partikel (8,9) sphärische Partikel sind, die in einer Kugelpackung angeordnet sind und die elektrisch leitfähigen Partikel (8) im Wesentlichen dieselbe Größe aufweisen,
- und die zwei elektrisch leitfähigen Partikel (8) über einer zwischen den vier Partikeln angeordneten Lücke gegeneinander elektrisch isoliert sind, wobei die Lücke eine Nanopore zur Nukleinsäure-Sequenzierung ausbildet.

2. Anordnung nach Anspruch 1, wobei die ersten Elektroden (4) jeweils mit wenigstens einem elektrisch leitenden Partikel (8) in direktem elektrischem Kontakt stehen.

3. Anordnung nach einem der vorangegangenen Ansprüche mit wenigstens zwei orthogonal zu den ersten Elektroden (4) angeordneten zweiten Elektroden (5).

4. Anordnung nach einem der vorangegangenen Ansprüche, wobei das Substrat (2) im Raster angeordnete Vertiefungen (3) umfasst.

5. Anordnung nach einem der vorangegangenen Ansprüche, wobei das Substrat (2) ein CMOS-Chip ist.

6. Anordnung nach einem der vorangegangenen Ansprüche, wobei die elektrisch leitfähigen Partikel (8) Gold umfassen.

7. Anordnung nach einem der vorangegangenen Ansprüche, wobei die elektrisch isolierenden Partikel (9) Polystyrol umfassen.

8. Anordnung nach einem der vorangegangenen Ansprüche, wobei die elektrisch leitfähigen Partikel (8), welche zueinander weniger als 1nm Abstand zueinander haben, fest miteinander verbunden sind.

9. Verfahren zum Herstellen einer Anordnung gemäß Anspruch 1 mit folgenden Schritten:
- Erzeugen von Vertiefungen (3) in einem Substrat,
- Zugeben einer Suspension umfassend wenigstens zwei elektrisch leitfähigen Partikeln (8) und wenigstens zwei elektrisch isolierenden Partikeln (9) in die Vertiefungen (3),
- wobei die Partikel (8,9) in den Vertiefungen (3) statistisch verteilt werden.

10. Verfahren nach Anspruch 9, wobei die Vertiefungen (3) mit wenigstens 100 Partikeln (8,9) befüllt werden.

11. Verfahren nach Anspruch 10, wobei die Vertiefungen mit statistisch verteilten Partikeln (8,9) selektiert werden, welche genau eine Anordnung gemäß Anspruch 1 aufweisen.

12. Verfahren nach einem der Anspruch 9 bis 11, bei dem die elektrisch leitfähigen Partikel (8), welche weniger als 1nm Abstand zueinander haben, mit einer elektrisch leitfähigen Schicht derart beschichtet werden, dass sie in elektrischem Kontakt miteinander stehen.

13. Verfahren nach Anspruch 12, bei dem die elektrisch leitfähigen Partikel (8) mittels stromlosen Abscheidens von Metall beschichtet werden.

## Claims

1. Arrangement for nucleic acid sequencing by means of tunneling current analysis comprising:
- at least two electrically conductive particles (8) having a diameter (11) of 1 nm to 100 nm,
- at least two electrically insulating particles (9) having a diameter of 1 nm to 100 nm,
- at least two first electrodes (4) for contacting the electrically conductive particles (8),
- a substrate (2), on which the first electrodes (4) and the particles (8, 9) are arranged,
- wherein the four particles (8, 9) are arranged in a substantially square planar fashion and the conductive particles (8) and the insulating particles (9) in each case lie diagonally opposite one another,
- wherein the particles (8, 9) are spherical particles which are arranged in a sphere packing and the electrically conductive particles (8) have substantially the same size,
- and the two electrically conductive particles (8) are electrically insulated from one another by means of a gap arranged between the four particles, wherein the gap forms a nanopore for nucleic acid sequencing.

2. Arrangement according to Claim 1, wherein the first electrodes (4) are in each case in direct electrical contact with at least one electrically conductive particle (8).

3. Arrangement according to either of the preceding claims comprising at least two second electrodes (5) arranged orthogonally with respect to the first electrodes (4).

4. Arrangement according to any of the preceding claims, wherein the substrate (2) comprises depressions (3) arranged in a grid.

5. Arrangement according to any of the preceding claims, wherein the substrate (2) is a CMOS chip.

6. Arrangement according to any of the preceding claims, wherein the electrically conductive particles (8) comprise gold.

7. Arrangement according to any of the preceding claims, wherein the electrically insulating particles (9) comprise polystyrene.

8. Arrangement according to any of the preceding claims, wherein the electrically conductive particles (8) which are at a distance from one another of less than 1 nm with respect to one another are fixedly connected to one another.

9. Method for producing an arrangement according to Claim 1, comprising the following steps:
- producing depressions (3) in a substrate,
- adding a suspension comprising at least two electrically conductive particles (8) and at least two electrically insulating particles (9) into the depressions (3),
- wherein the particles (8, 9) are statistically distributed in the depressions (3).

10. Method according to Claim 9, wherein the depressions (3) are filled with at least 100 particles (8, 9).

11. Method according to Claim 10, wherein the depressions with statistically distributed particles (8, 9) are selected which have exactly an arrangement according to Claim 1.

12. Method according to any of Claims 9 to 11, wherein the electrically conductive particles (8) which are at a distance from one another of less than 1 nm are coated with an electrically conductive layer in such a way that they are in electrical contact with one another.

13. Method according to Claim 12, wherein the electrically conductive particles (8) are coated by means of electroless deposition of metal.

## Revendications

1. Système de séquençage d'acides nucléiques au moyen d'une analyse par courant tunnel, comprenant :
- au moins deux particules (8) conductrices de l'électricité d'un diamètre (11) de 1 nm à 100 nm,
- au moins deux particules (9) isolantes du point de vue électrique d'un diamètre de 1 nm à 100 nm,
- au moins deux premières électrodes (4) pour entrer en contact avec les particules (8) conductrices de l'électricité,
- un substrat (2) sur lequel sont mises les premières électrodes (4) et les particules (8, 9),
- dans lequel les quatre particules (8, 9) sont disposés de manière plane, sensiblement suivant un carré, et les particules (8) conductrices et les particules (9) isolantes se font face respectivement en diagonale,
- dans lequel les particules (8, 9) sont des particules sphériques, qui sont disposées suivant un empilement de sphères et les particules (8) conductrices de l'électricité ont sensiblement la même dimension,
- et les deux particules (8) conductrices de l'électricité sont isolées, l'une par rapport à l'autre, par une lacune entre les quatre particules, la lacune constituant un nanoport pour le séquençage d'acides nucléiques.

2. Système suivant la revendication 1, dans lequel les premières électrodes (4) sont chacune en contact électrique direct avec au moins une particule (8) conductrice de l'électricité.

3. Système suivant l'une des revendications précédentes, ayant au moins deux deuxièmes électrodes (5) disposées orthogonalement aux premières électrodes (4).

4. Système suivant l'une des revendications précédentes, dans lequel le substrat (2) comprend des cavités (3) disposées en trame.

5. Système suivant l'une des revendications précédentes, dans lequel le substrat (2) est une puce CMOS.

6. Système suivant l'une des revendications précédentes, dans lequel les particules (8) conductrices de l'électricité comprennent de l'or.

7. Système suivant l'une des revendications précédentes, dans lequel les particules (9) isolantes du point de vue électrique comprennent du polystyrène.

8. Système suivant l'une des revendications précédentes, dans lequel les particules (8) conductrices de l'électricité, qui ont entre elles une distance inférieure à 1 nm, sont reliées fixement entre elles.

9. Procédé de production d'un système suivant la revendication 1, comprenant les stades suivantes :
- production de cavité (3) dans un substrat,
- versement d'une suspension, comprenant au moins deux particules (8) conductrices de l'électricité et au moins deux particules (9) isolantes électriquement, dans les cavités (3),
- les particules (8, 9) étant réparties statistiquement dans les cavités (3).

10. Procédé suivant la revendication 9, dans lequel on remplit les cavités (3) d'au moins 100 particules (8, 9).

11. Procédé suivant la revendication 10, dans lequel on sélectionne les cavités ayant des particules (8, 9) réparties statistiquement, qui ont exactement un système suivant la revendication 1.

12. Procédé suivant l'une des revendications 9 à 11, dans lequel on revêt les particules (8) conductrices de l'électricité, qui ont entre elles une distance de moins de 1 nm, d'une couche conductrice de l'électricité, de manière à ce qu'elles soient en contact électrique les unes avec les autres.

13. Procédé suivant la revendication 12, dans lequel on revêt les particules (8) conductrices de l'électricité au moyen d'un dépôt de métal sans courant.
